# EUROPÄISCHE PATENTSCHRIFT

(11) **EP 2 448 904 B1**
(45) Veröffentlichungstag und Bekanntmachung des Hinweises auf die Patenterteilung: **17.04.2013**
(21) Anmeldenummer: 10723943.6
(22) Anmeldetag: 09.06.2010
(51) Int. Cl.: C07C 67/08, C07D 213/79, C08L 67/02, C07C 69/78, C07C 69/76, C07C 69/88, C07C 69/82, B01J 19/12, H05B 6/70, H05B 6/80

(54) **KONTINUIERLICHES VERFAHREN ZUR HERSTELLUNG VON ESTERN AROMATISCHER CARBONSÄUREN**
CONTINUOUS METHOD FOR PRODUCING ESTERS OF AROMATIC CARBOXYLIC ACIDS
PROCÉDÉ DE PRODUCTION CONTINUE D'ESTERS D'ACIDES CARBOXYLIQUES AROMATIQUES

(30) Priorität: 30.06.2009 DE 102009031054
(43) Veröffentlichungstag der Anmeldung: 09.05.2012
(73) Patentinhaber: Clariant Finance (BVI) Limited, Road Town, Tortola (VG)
(72) Erfinder: KRULL, Matthias, 55296 Harxheim (DE); MORSCHHÄUSER, Roman, 55122 Mainz (DE)
(74) Vertreter: Mikulecky, Klaus
(86) Internationale Anmeldenummer: PCT/EP2010/003447
(87) Internationale Veröffentlichungsnummer: WO 2011/000464

(56) Entgegenhaltungen:
- WO-A1-2008/043493
- US-A- 5 387 397
- NOEL S WILSON ET AL: "Development and Applications of a Practical Continuous Flow Microwave Cell", ORGANIC PROCESS RESEARCH AND DEVELOPMENT, AMERICAN CHEMICAL SOCIETY, US, Bd. 8, Nr. 3, 1. Januar 2004 (2004-01-01), Seiten 535-538, XP007916928, ISSN: 1083-6160, DOI: DOI:10.1021/OP034181B [gefunden am 2004-01-23] in der Anmeldung erwähnt
- CABLEWSKI T ET AL: "DEVELOPMENT AND APPLICATION OF A CONTINUOUS MICROWAVE REACTOR FOR ORGANIC SYNTHESIS", JOURNAL OF ORGANIC CHEMISTRY, AMERICAN CHEMICAL SOCIETY, EASTON.; US, Bd. 59, 1. Januar 1994 (1994-01-01), Seiten 3408-3412, XP000198783, ISSN: 0022-3263, DOI: DOI:10.1021/JO00091A033
- PIPUS G ET AL: "Esterification of benzoic acid in microwave tubular flow reactor", CHEMICAL ENGINEERING JOURNAL, ELSEVIER SEQUOIA, LAUSANNE, CH, Bd. 76, 1. Januar 2000 (2000-01-01), Seiten 239-245, XP007916929, ISSN: 1385-8947

## Beschreibung

Die vorliegende Erfindung betrifft ein kontinuierliches Verfahren zur Herstellung von Estern aromatischer Carbonsäuren unter Mikrowellenbestrahlung im technischen Maßstab.

Ester sind eine industriell sehr wichtige Stoffgruppe, die vielfältige Verwendung findet und beispielsweise als Weichmacher, Schmierstoff sowie als Bestandteil von Kosmetika und Pharmazeutika eingesetzt wird. Eine bewährte und vielfach angewendete Methode zur Herstellung von Estern ist die Kondensation von Carbonsäuren mit Alkoholen in Gegenwart von Katalysatoren. Dabei wird das Reaktionsgemisch gewöhnlich mehrere Stunden lang erhitzt und das sich bildende Wasser ausgetragen. Es sind auch Verfahren bekannt, bei denen die Veresterung in einem geschlossenen System unter Druck und hohen Temperaturen durchgeführt wird. So offenbart WO 2007/126166 eine konventionell thermische Veresterung von Fettsäuren mit Alkoholen bei Temperaturen von 200 bis 350 °C und Drucken von bis zu 10 bar. Bei der mehrstündigen Reaktion, in deren Verlauf das entstehende Reaktionswasser kontinuierlich mit überschüssigem Alkohol ausgetragen wird, wird jedoch nur eine unvollständige Umsetzung zum Methylester erreicht, so dass eine aufwendige Aufarbeitung bzw. Weiterverarbeitung des Rohprodukts erforderlich ist. Problematisch bei derartigen Hochtemperaturreaktionen ist weiterhin die Korrosivität der Reaktionsgemische, die einerseits zur Schädigung der Reaktionsgefäße und andererseits zu unerwünschten Metallgehalten in den so hergestellten Estern führt.

Ein neuerer Ansatz zur Synthese von Estern ist die durch Mikrowellen unterstützte Umsetzung von Carbonsäuren und Alkoholen, wodurch insbesondere die für befriedigende Ausbeuten erforderlichen Reaktionszeiten deutlich vermindert werden.

Q. Yang et al. (Synth. Commun. 2008, 38, 4107-4115) beschreiben sauer katalysierte Veresterungen verschiedener Carbonsäuren mit Alkoholen unter Mikrowellenbestrahlung. Die Reaktionen werden bei 100 °C im Labormaßstab durchgeführt und führen zu hohen Umsätzen.

US 2005/0274065 offenbart ein Verfahren, bei dem Fettsäuren mit Alkoholen in Gegenwart von Katalysatoren und/oder unter dem Einfluss von Mikrowellenenergie verestert werden. Dabei wird in einer speziellen Ausführungsform das sich in einer Vorlage befindliche Reaktionsgut kontinuierlich umgepumpt und dabei durch einen in einem Mikrowellen-Applikator befindlichen, gerührten Behälter geführt. Erst nach mehrmaligem Durchfahren des MikrowellenApplikators werden hohe Veresterungsgrade erzielt.

Das Scale-Up derartiger mikrowellenunterstützter Reaktionen aus dem Labor in einen technischen Maßstab und damit die Entwicklung von Anlagen, die für eine Produktion von mehreren Tonnen wie beispielsweise mehreren zehn, mehreren hundert oder mehreren tausend Tonnen pro Jahr mit für großtechnische Anwendungen interessanten Raum-Zeit-Ausbeuten geeignet sind, konnte bisher jedoch nicht realisiert werden. Ursache dafür ist zum einen die üblicherweise auf einige Millimeter bis wenige Zentimeter begrenzte Eindringtiefe von Mikrowellen in das Reaktionsgut, was insbesondere in Batch-Verfahren durchgeführte Reaktionen auf kleine Gefäße beschränkt oder in gerührten Reaktoren zu sehr langen Reaktionszeiten führt. Einer für die Bestrahlung großer Substanzmengen mit Mikrowellen wünschenswerten Erhöhung der Feldstärke sind insbesondere in den bisher bevorzugt zum Scale-Up chemischer Reaktionen eingesetzten Multimode-Geräten durch dann auftretende Entladungsvorgänge und Plasmabildung enge Grenzen gesetzt. Weiterhin bereitet die in Multimode-Mikrowellengeräten zu lokalen Überhitzungen des Reaktionsgutes führende, durch mehr oder weniger unkontrollierte Reflektionen der in den Mikrowellenofen eingestrahlten Mikrowellen an dessen Wänden und dem Reaktionsgut verursachte Inhomogenität des Mikrowellenfeldes Probleme bei der Maßstabsvergrößerung. Zudem bereitet der sich während der Reaktion oftmals ändernde Mikrowellen-Absorptionskoeffizient des Reaktionsgemischs Schwierigkeiten hinsichtlich einer sicheren und reproduzierbaren Reaktionsführung.

WO 90/03840 offenbart ein kontinuierliches Verfahren zur Durchführung verschiedener chemischer Reaktionen wie beispielsweise Veresterungen in einem kontinuierlichen Labor-Mikrowellenreaktor. Die erzielten Ausbeuten wie auch das Reaktionsvolumen von 24 ml der im Multimode betriebenen Mikrowelle erlauben jedoch kein Up-Scaling in den großtechnischen Bereich. Der Wirkungsgrad dieses Verfahrens bezüglich der Mikrowellenabsorption des Reaktionsguts ist auf Grund der in Multimode-Mikrowellenapplikatoren auf den Applikatorraum mehr oder weniger homogen verteilten und nicht auf die Rohrschlange fokussierten Mikrowellenenergie niedrig. Eine starke Erhöhung der eingestrahlten Mikrowellenleistung kann zu unerwünschten Plasma- Entladungen oder zu so genannten thermischen Runaway-Effekten führen. Weiterhin machen die als Hot-Spots bezeichneten, sich zeitlich verändernden räumlichen Inhomogenitäten des Mikrowellenfeldes im Applikatorraum eine sichere und reproduzierbare Reaktionsführung im großen Maßstab unmöglich.

Weiterhin sind aus Kappe et al., Top. Curr. Chem. (2006) 266: 233 - 276, Monomode- bzw. Singlemode-Mikrowellenapplikatoren bekannt, bei denen mit einem einzigen Wellen-Modus gearbeitet wird, der sich in nur einer Raumrichtung ausbreitet und durch exakt dimensionierte Wellenleiter auf das Reaktionsgefäß fokussiert wird. Diese Geräte erlauben zwar höhere lokale Feldstärken, sind bisher aber auf Grund der geometrischen Anforderungen (z. B. ist die Intensität des elektrischen Feldes an seinen Wellenbergen am größten und geht an den Knotenpunkten gegen Null) auf kleine Reaktionsvolumina (≤ 50 ml) im Labormaßstab beschränkt.

WO-2008/043493 offenbart ein Verfahren zur Synthese von Fettsäurealkanolamiden durch kontinuierliche Amidierung von Fettsäuren unter Einsatz von Mikrowellen. Die Mikrowellen werden dabei in einer Ausführungsform mittels mindestens einer Antenne über die Rohrenden eingestrahlt. Den Unterschied des daraus bekannten Verfahrens gegenüber dem erfindungsgemäßen Verfahren besteht darin, dass statt der erfindungsgemäßen Aminosäure dort ein Aminoalkohol beschrieben ist.

So beschreiben Chemat et al. (J. Microwave Power and Electromagnetic Energy 1998, 33, 88-94) verschiedene kontinuierlich durchgeführte Veresterungen in einer Monomode-Mikrowellenkavität, wobei der Mikrowellenleiter senkrecht auf das Reaktionsrohr trifft. Dabei werden bei heterogen katalysierten Veresterungen beschleunigte Umsetzungen beobachtet. Das für die Mikrowellenbestrahlung zur Verfügung stehende Volumen von nur 20 ml erfordert jedoch mehrmaliges Durchfahren der Reaktanden durch die Bestrahlungszone, um interessante Ausbeuten zu erzielen. Eine deutliche Erhöhung des Querschnitts des Reaktionsrohres ist auf Grund der Geometrie des Applikators nicht möglich und wegen der geringen Eindringtiefe von Mikrowellen auch nicht zum Up-Scaling geeignet.

Pipus et al. (First European Congress on Chemical Engineering, Firenze, Italy, May 4-7, 1997; AIDIC: Milan, Italy, 1997; pp. 45-48) offenbaren homogen wie auch heterogen katalysierte Veresterungen von Benzoesäure mit Ethanol in einem kontinuierlichen, mit Mikrowellenstrahlung geheizten Rohrreaktor. Bei einem Druck von 7 atm und einer Temperatur von 140 °C wird bei einer Verweilzeit im Reaktor von 127 Sekunden ein Umsatz von 30 % erreicht.

Wilson et al. (Org. Process Res. Dev. 2004, 8, 535-538) offenbaren einen kontinuierlichen Mikrowellenreaktor, in dem eine Mischung aus 2,4,6-Trimethylbenzosäure und Methanol in einer Glasschlange mit einem Volumen von 4 ml durch ein monomodales Mikrowellenfeld geführt und dabei verestert wird. Auf Grund der geometrischen Bedingungen dieses Aufbaus ist das Verfahren nicht auf großtechnische Anwendungen übertragbar.

Es wurde daher ein Verfahren zur Herstellung von Estern aromatischer Carbonsäuren gesucht, bei dem aromatische Carbonsäure und Alkohol auch im technischen Maßstab unter Mikrowellenbestrahlung zum Ester umgesetzt werden können. Dabei sollen möglichst hohe, das heißt bis zu quantitative Umsetzungsraten und Ausbeuten erzielt werden. Das Verfahren soll weiterhin eine möglichst energiesparende Herstellung der Ester ermöglichen, das heißt, die eingesetzte Mikrowellenleistung soll möglichst quantitativ vom Reaktionsgut absorbiert werden und das Verfahren somit einen hohen energetischen Wirkungsgrad bieten. Dabei sollen keine bzw. nur untergeordnete Mengen an Nebenprodukten anfallen. Die Ester sollen ferner einen möglichst niedrigen Metallgehalt und eine geringe Eigenfärbung aufweisen. Zudem soll das Verfahren eine sichere und reproduzierbare Reaktionsführung gewährleisten.

Überraschenderweise wurde gefunden, dass sich Ester aromatischer Carbonsäuren durch direkte Umsetzung von aromatischen Carbonsäuren mit Alkoholen in einem kontinuierlichen Verfahren durch nur kurzzeitiges Erhitzen mittels Bestrahlung mit Mikrowellen in einem Reaktionsrohr, dessen Längsachse sich in der Ausbreitungsrichtung der Mikrowellen eines Monomode-Mikrowellenapplikators befindet, in technisch relevanten Mengen herstellen lassen. Dabei wird die in den Mikrowellenapplikator eingestrahlte Mikrowellenenergie praktisch quantitativ vom Reaktionsgut absorbiert. Das erfindungsgemäße Verfahren besitzt zudem eine hohe Sicherheit bei der Durchführung und bietet eine hohe Reproduzierbarkeit der eingestellten Reaktionsbedingungen. Die nach dem erfindungsgemäßen Verfahren hergestellten Ester zeigen eine im Vergleich zu nach konventionellen Herstellverfahren ohne zusätzliche Verfahrensschritte nicht zugängliche hohe Reinheit und niedrige Eigenfärbung.

Gegenstand der Erfindung ist ein kontinuierliches Verfahren zur Herstellung von Carbonsäureestern, in dem mindestens eine aromatische Carbonsäure der Formel (I)

Ar-COOH (I)

worin Ar für einen gegebenenfalls substituierten Arylrest mit 5 bis 50 Atomen steht,
mit mindestens einem Alkohol der Formel (II)

R²-(OH)ₙ (II)

worin
- R²: für einen gegebenenfalls substituierten Kohlenwasserstoffrest mit 1 bis 100 C-Atomen und
- n: für eine Zahl von 1 bis 10 stehen,
in Gegenwart mindestens eines Veresterungskatalysators unter Mikrowellenbestrahlung in einem Reaktionsrohr, dessen Längsachse sich in der Ausbreitungsrichtung der Mikrowellen eines Monomode-Mikrowellenapplikators befindet, zum Ester umgesetzt wird, bei dem die Bestrahlung des Reaktionsgemischs mit Mikrowellen in einem weitgehend mikrowellentransparenten Reaktionsrohr innerhalb eines mit einem Mikrowellengenerator über Wellenleiter verbundenen Hohlleiters erfolgt.

Ar ist bevorzugt ein Arylrest, der mindestens eine an ein aromatisches System gebundene Carboxylgruppe trägt. Unter aromatischen Systemen werden zyklische, durchkonjugierte Systeme mit (4 n + 2) π-Elektronen verstanden, worin n eine natürliche ganze Zahl und vorzugsweise 1, 2, 3, 4 oder 5 ist. Das aromatische System kann mono- oder polyzyklisch wie beispielsweise di- oder trizyklisch sein. Bevorzugt hat das aromatische System 6 bis 25 Atome, besonders bevorzugt 6 bis 18 Atome. Das aromatische System wird bevorzugt aus Kohlenstoffatomen gebildet. In einer weiteren bevorzugten Ausführungsform enthält es neben Kohlenstoffatomen ein oder mehrere Heteroatome wie beispielsweise Stickstoff, Sauerstoff und/oder Schwefel. Beispiele für solche aromatischen Systeme sind Benzol, Naphthalin, Indol, Phenanthren, Pyridin, Furan, Pyrrol, Thiophen und Thiazol. Das aromatische System kann neben der Carboxylgruppe ein oder mehrere wie beispielsweise eins, zwei, drei oder mehr gleiche oder verschiedene weitere Substituenten tragen. Geeignete weitere Substituenten sind beispielsweise Alkyl-, Alkenyl- und halogenierte Alkylreste, Hydroxy-, Hydroxyalkyl-, Alkoxy-, Poly(alkoxy)-, Halogen-, Carboxyl-, Amid-, Cyano-, Nitril- und/oder Nitrogruppen. Diese Substituenten können an beliebiger Position des aromatischen Systems gebunden sein. Der Arylrest trägt jedoch höchstens so viele Substituenten, wie er Valenzen hat.

In einer speziellen Ausführungsform trägt der Arylrest Ar der Formel (I) weitere Carboxylgruppen. So ist das erfindungsgemäße Verfahren ebenso zur Umsetzung von aromatischen Carbonsäuren mit beispielsweise zwei oder mehr Carboxylgruppen geeignet. Das Ausmaß der Veresterung kann beispielsweise über die Stöchiometrie zwischen Säure (I) und Alkohol (II) im Reaktionsgemisch gesteuert werden.

Besonders geeignet ist das erfindungsgemäße Verfahren zur Veresterung von Alkylarylcarbonsäuren wie beispielsweise Alkylphenylcarbonsäuren. Dabei handelt es sich um aromatische Carbonsäuren, bei denen der die Carboxylgruppe tragende Arylrest Ar zusätzlich mindestens einen Alkyl- oder Alkenylrest trägt. Besonders vorteilhaft ist das Verfahren bei der Veresterung von Alkylbenzoesäuren, die mindestens einen Alkylrest mit 1 bis 50 C-Atomen, bevorzugt mit 2 bis 20 C-Atomen und insbesondere 1 bis 12 C-Atomen wie beispielsweise 1 bis 4 C-Atomen tragen.

Weiterhin besonders geeignet ist das erfindungsgemäße Verfahren zur Veresterung von aromatischen Carbonsäuren, deren Arylrest Ar eine oder mehrere wie beispielsweise zwei, drei oder mehr Hydroxylgruppen und/oder Hydroxyalkylgruppen trägt. Bei der Veresterung insbesondere mit mindestens equimolaren Mengen an Alkohol der Formel (II) findet dabei selektiv eine Veresterung der Carboxylgruppe statt; es findet keine Veresterung der phenolischen OH-Gruppe unter Bildung von Polyestern statt.

Geeignete aromatische Carbonsäuren sind beispielsweise Benzoesäure, Phthalsäure, Isophthalsäure, Terephthalsäure, die verschiedenen Isomeren der Naphthalincarbonsäure, der Pyridincarbonsäure und der Naphthalindicarbonsäure sowie Trimellitsäure, Trimesinsäure, Pyromellitsäure und Mellitsäure, die verschiedenen Isomere der Methoxybenzoesäure, Hydroxybenzoesäure, Hydroxymethylbenzoesäure, Hydroxymethoxybenzoesäure, Hydroxydimethoxybenzoesäure, Hydroxyisophthalsäure, Hydroxynaphthalincarbonsäure, Hydoxypyridincarbonsäure und Hydroxymethylpyridincarbonsäure, Hydroxychinolincarbonsäure sowie o-Tolylsäure, m-Tolylsäure, p-Tolylsäure, o-Ethylbenzoesäure, m-Ethylbenzoesäure, p-Ethylbenzoesäure, o-Propylbenzoesäure, m-Propylbenzoesäure, p-Propylbenzoesäure, 3,4-Dimethylbenzoesäure, und Thiophencarbonsäure. Mischungen verschiedener Aryl- und/oder Alkylarycarbonsäuren sind gleichfalls geeignet.

In einer bevorzugten Ausführungsform steht R² für einen aliphatischen Rest. Dieser hat bevorzugt 1 bis 24, besonders bevorzugt 2 bis 18 und speziell 3 bis 6 C-Atome. Der aliphatische Rest kann linear, verzweigt oder zyklisch sein. Er kann weiterhin gesättigt oder ungesättigt sein, bevorzugt ist er gesättigt. Der Kohlenwasserstoffrest kann Substituenten wie beispielsweise Halogenatome, halogenierte Alkylreste, Hydroxyl-, C₁-C₅-Alkoxyalkyl-, Cyano-, Nitril-, Nitro- und/oder C₅-C₂₀-Arylgruppen wie beispielsweise Phenylreste tragen. Die C₅-C₂₀-Arylreste können ihrerseits gegebenenfalls mit Halogenatomen, halogenierten Alkylresten, Hydroxyl-, C₁-C₂₀-Alkyl-, C₂-C₂₀-Alkenyl-, C₁-C₅-Alkoxy- wie beispielsweise Methoxy-, Ester-, Amid-, Cyano-, Nitril-, und/oder Nitrogruppen substituiert sein. Besonders bevorzugte aliphatische Reste sind Methyl, Ethyl, n-Propyl, iso-Propyl, n-Butyl, iso-Butyl und tert.-Butyl, n-Hexyl, Cyclohexyl, n-Octyl, n-Decyl, n-Dodecyl, Tridecyl, Isotridecyl, Tetradecyl, Hexadecyl, Octadecyl und Methylphenyl.

In einer weiteren bevorzugten Ausführungsform steht R² für eine gegebenenfalls substituierte C₆-C₁₂-Arylgruppe oder eine gegebenenfalls substituierte heteroaromatische Gruppe mit 5 bis 12 Ringgliedern. Bevorzugte Heteroatome sind Sauerstoff, Stickstoff und Schwefel. An die C₆-C₁₂-Arylgruppe bzw. die heteroaromatische Gruppe mit 5 bis 12 Ringgliedern können weitere Ringe annelliert sein. Die Aryl- bzw. heteroaromatische Gruppe kann somit mono- oder polyzyklisch sein. Beispiele für geeignete Substituenten sind Halogenatome, halogenierte Alkylreste sowie Alkyl-, Alkenyl-, Hydroxy-, Hydroxyalkyl-, Alkoxy-, Ester-, Amid-, Nitril- und Nitrogruppen.

In einer speziellen Ausführungsform trägt der Rest R² eine oder mehrere wie beispielsweise zwei, drei, vier, fünf, sechs oder mehr weitere Hydroxylgruppen, jedoch nicht mehr Hydroxylgruppen als der Rest R² C-Atome bzw. als die Arylgruppe Valenzen besitzt. Die Hydroxylgruppen können an benachbarten C-Atomen oder auch an weiter entfernten Kohlenstoffatomen des Kohlenwasserstoffrestes gebunden sein, jedoch höchstens eine OH-Gruppe pro Kohlenstoffatom. So eignet sich das erfindungsgemäße Verfahren auch zur Veresterung von Polyolen wie beispielsweise Ethylenglykol, 1,2-Propandiol, 1,3-Propandiol, Neopentylglykol, Glycerin, Sorbit, Pentaerythrit, Fructose und Glucose. Die Veresterung kann dabei zu Vollestern oder auch Partialestern geführt werden. Der Veresterungsgrad lässt sich dabei beispielsweise über die Stöchiometrie zwischen Carbonsäure und Alkohol im Reaktionsgemisch steuern.

Für den Fall, dass die Carbonsäure (I) zwei oder mehr Carboxylgruppen und der Alkohol (II) zwei oder mehr Hydroxylgruppen enthält bzw. beide Reaktanden jeweils mindestens eine Carboxyl- und mindestens eine Hydroxylgruppe tragen, können gemäß dem erfindungsgemäßen Verfahren auch Oligomere und Polymere hergestellt werden. Bei derartigen Polykondensationen ist die während der Mikrowellenbestrahlung ansteigende Viskosität des Reaktionsgemischs bei der Auslegung der Apparatur zu beachten.

In einer weiteren bevorzugten Ausführungsform steht R² für einen mit Heteroatomen unterbrochenen-Alkylrest. Besonders bevorzugte Heteroatome sind Sauerstoff und Stickstoff. Sofern der Rest R² Stickstoffatome enthält, so tragen diese Stickstoffatome jedoch keine aciden Protonen.

So steht R² bevorzugt für Reste der Formel (III)

-(R⁴-O)ₙ-R⁵ (III)

worin
- R⁴: für eine Alkylengruppe mit 2 bis 18 C-Atomen, bevorzugt mit 2 bis 12 und insbesondere 2 bis 4 C-Atomen wie beispielsweise Ethylen, Propylen, Butylen oder Mischungen daraus,
- R⁵: für Wasserstoff oder einen Kohlenwasserstoffrest mit 1 bis 24 C-Atomen oder eine Gruppe der Formel -R⁴-NR¹⁰R¹¹,
- n: für eine Zahl zwischen 1 und 500, bevorzugt zwischen 2 und 200 und insbesondere zwischen 3 und 50 wie beispielsweise zwischen 4 und 20 und
- R¹⁰, R¹¹: unabhängig voneinander für einen aliphatischen Rest mit 1 bis 24 C-Atomen und bevorzugt 2 bis 18 C-Atomen, eine Arylgruppe- oder Heteroarylgruppe mit 5 bis 12 Ringgliedern, eine Poly(oxyalkylen)gruppe mit 1 bis 50 Poly(oxyalkylen)einheiten, wobei sich die Polyoxyalkyleneinheiten von Alkylenoxideinheiten mit 2 bis 6 C-Atomen ableiten, oder R¹⁰ und R¹¹ gemeinsam mit dem Stickstoffatom, an das sie gebunden sind, einen Ring mit 4, 5, 6 oder mehr Ringgliedern bilden, stehen.

Beispiele für geeignete Alkohole sind Methanol, Ethanol, n-Propanol, iso-Propanol, n-Butanol, iso-Butanol, tert.-Butanol, Pentanol, Neopentanol, n-Hexanol, iso-Hexanol, Cyclohexanol, Heptanol, Octanol, Decanol, Dodecanol, Tetradecanol, Hexadecanol, Octadecanol, Eicosanol, Ethylenglykol, 2-Methoxyethanol, Propylenglykol, Diethylenglykol, Triethylenglykol, Polyethylenglykol, Polypropylenglykol, Triethanolamin, N,N-Dimethylethanolamin, N,N-Diethylethanolamin, Phenol, Naphthol und deren Mischungen. Weiterhin geeignet sind aus natürlichen Rohstoffen gewonnene Fettalkoholmischungen wie beispielsweise Cocosfettalkohol, Palmkernfettalkohol und Talgfettalkohol.

Das Verfahren ist insbesondere geeignet zur Herstellung von Benzoesäuremethylester, Benzoesäureethylester, Benzoesäure-n-hexylester, Benzoesäure-o-tolylester, p-Tolylsäuremethylester, p-Hydroxybenzoesäuremethylester, p-Hydroxybenzoesäureethylester, Nicotinsäuremethylester, Nicotinsäureethylester, Nicotinsäurephenylester.

Für den Fall, dass die Carbonsäure (I) zwei oder mehr Carboxylgruppen und der Alkohol (II) zwei oder mehr Hydroxylgruppen enthält, können gemäß dem erfindungsgemäßen Verfahren auch Polymere hergestellt werden. Dabei ist die während der Mikrowellenbestrahlung ansteigende Viskosität des Reaktionsgemischs bei der Auslegung der Apparatur zu beachten.

Im erfindungsgemäßen Verfahren können aromatische Carbonsäure (I) und Alkohol (II) in beliebigen Verhältnissen miteinander zur Reaktion gebracht werden. Bevorzugt erfolgt die Umsetzung zwischen Carbonsäure und Alkohol mit molaren Verhältnissen von 20:1 bis 1:20, bevorzugt von 10:1 bis 1:10 und speziell von 3:1 bis 1:3 wie beispielsweise von 1,5:1 bis 1:1,5, jeweils bezogen auf die Molequivalente an Carboxyl- und Hydroxylgruppen. In einer speziellen Ausführungsform werden Carbonsäure und Alkohol equimolar eingesetzt. Sofern die aromatische Carbonsäure (I) eine oder mehrere Hydroxylgruppen trägt, erfolgt die Umsetzung bevorzugt mit mindestens equimolaren Anteilen Alkohol (II), besonders bevorzugt im Verhältnis von aromatischer Carbonsäure (I) zu Alkohol (II) von 1:1,01 bis 1:50, speziell im Verhältnis 1:1,5 bis 1:20 wie beispielsweise 1:2 bis 1:10.

In vielen Fällen hat es sich als vorteilhaft erwiesen, mit einem Überschuss an Alkohol, das heißt molaren Verhältnissen von Hydroxyl- zu Carboxylgruppen von mindestens 1,01:1,00 und insbesondere zwischen 50:1 und 1,02:1 wie beispielsweise zwischen 10:1 und 1,1:1 zu arbeiten. Dabei werden die Carboxylgruppen praktisch quantitativ zum Ester umgesetzt. Besonders vorteilhaft ist dieses Verfahren, wenn der eingesetzte Alkohol leicht flüchtig ist. Leicht flüchtig heißt hier, dass der Alkohol einen Siedepunkt bei Normaldruck von vorzugsweise unterhalb 200 °C und besonders bevorzugt unterhalb 160 °C wie beispielsweise unterhalb 100 °C besitzt und sich somit destillativ vom Ester abtrennen lässt.

Die Veresterungen werden im erfindungsgemäßen Verfahren in Gegenwart von homogenen Katalysatoren, heterogenen Katalysatoren oder deren Mischungen durchgeführt. Sowohl saure wie auch alkalische Katalysatoren sind dabei geeignet. Erfindungsgemäß bevorzugte Veresterungskatalysatoren sind saure anorganische, metallorganische oder organische Katalysatoren und Gemische aus mehreren dieser Katalysatoren.

Als saure anorganische Katalysatoren im Sinne der vorliegenden Erfindung sind beispielsweise Schwefelsäure, Phosphorsäure, Phosphonsäure, hypophosphorige Säure, Aluminiumsulfathydrat, Alaun, saures Kieselgel und saures Aluminiumhydroxid zu nennen. Weiterhin sind beispielsweise Aluminiumverbindungen der allgemeinen Formel Al(OR¹⁵)₃ und Titanate der allgemeinen Formel Ti(OR¹⁵)₄ als saure anorganische Katalysatoren einsetzbar, wobei die Reste R¹⁵ jeweils gleich oder verschieden sein können und unabhängig voneinander gewählt sind aus C₁-C₁₀-Alkylresten, beispielsweise Methyl, Ethyl, n-Propyl, iso-Propyl, n-Butyl, iso-Butyl, sec.-Butyl, tert.-Butyl, n-Pentyl, iso-Pentyl, sec.-Pentyl, neo-Pentyl, 1,2-Dimethylpropyl, iso-Amyl, n-Hexyl, sec.-Hexyl, n-Heptyl, n-Octyl, 2-Ethylhexy, n-Nonyl oder n-Decyl, C₃-C₁₂-Cycloalkylresten, beispielsweise Cyclopropyl, Cyclobutyl, Cyclopentyl, Cyclohexyl, Cycloheptyl, Cyclooctyl, Cyclononyl, Cyclodecyl, Cycloundecyl und Cyclododecyl; bevorzugt sind Cyclopentyl, Cyclohexyl und Cycloheptyl. Bevorzugt sind die Reste R¹⁵ in Al(OR¹⁵)₃ bzw. Ti(OR¹⁵)₄ jeweils gleich und gewählt aus Isopropyl, Butyl und 2-Ethylhexyl.

Bevorzugte saure metallorganische Katalysatoren sind beispielsweise gewählt aus Dialkylzinnoxiden (R¹⁵)₂SnO, wobei R¹⁵ wie oben stehend definiert ist. Ein besonders bevorzugter Vertreter für saure metallorganische Katalysatoren ist Di-n-butylzinnoxid, das als sogenanntes Oxo-Zinn oder als Fascat^{®}-Marken kommerziell erhältlich ist.

Bevorzugte saure organische Katalysatoren sind saure organische Verbindungen mit beispielsweise Phosphatgruppen, Sulfonsäuregruppen, Sulfatgruppen oder Phosphonsäuregruppen. Besonders bevorzugte Sulfonsäuren enthalten mindestens eine Sulfonsäuregruppe und mindestens einen gesättigten oder ungesättigten, linearen, verzweigten und/oder zyklischen Kohlenwasserstoffrest mit 1 bis 40 C-Atomen und bevorzugt mit 3 bis 24 C-Atomen. Insbesondere bevorzugt sind aromatische Sulfonsäuren und speziell alkylaromatische Mono-Sulfonsäuren mit einem oder mehreren C₁-C₂₈-Alkylresten und insbesondere solche mit C₃-C₂₂-Alkylresten. Geeignete Beispiele sind Methansulfonsäure, Butansulfonsäure, Benzolsulfonsäure, p-Toluolsulfonsäure, Xylolsulfonsäure, 2-Mesitylensulfonsäure, 4-Ethylbenzolsulfonsäure, Isopropylbenzolsulfonsäure, 4-Butylbenzolsulfonsäure, 4-Octylbenzolsulfonsäure; Dodecylbenzolsulfonsäure, Didodecylbenzolsulfonsäure, Naphthalinsulfonsäure. Auch saure Ionenaustauscher können als saure organische Katalysatoren eingesetzt werden, beispielsweise Sulfonsäuregruppen tragende Poly(styrol)-Harze, die mit etwa 2 mol-% Divinylbenzol vernetzt sind.

Besonders bevorzugt für die Durchführung des erfindungsgemäßen Verfahrens sind Borsäure, Phosphorsäure, Polyphosphorsäure und Polystyrolsulfonsäuren. Insbesondere bevorzugt sind Titanate der allgemeinen Formel Ti(OR¹⁵)₄ und speziell Titantetrabutylat und Titantetraisopropylat.

Wünscht man saure anorganische, metallorganische oder organische Katalysatoren einzusetzen, so setzt man erfindungsgemäß 0,01 bis 10 Gew.-%, bevorzugt 0,02 bis 2 Gew.-% Katalysator ein.

In einer weiteren bevorzugten Ausführungsform wird die Mikrowellenbestrahlung in Gegenwart von sauren, festen Katalysatoren durchgeführt. Derartige heterogene Katalysatoren können im Reaktionsgemisch suspendiert und gemeinsam mit dem Reaktionsgemisch durch das Reaktionsrohr gepumpt werden. In einer besonders bevorzugten Ausführungsform wird das gegebenenfalls mit Lösemittel versetzte Reaktionsgemisch über einen im Reaktionsrohr fixierten Festbettkatalysator geleitet und dabei Mikrowellenstrahlung ausgesetzt. Geeignete feste Katalysatoren sind beispielsweise Zeolithe, Kieselgel, Montmorillonit und (teil)vernetzte Polystyrolsulfonsäure, die gegebenenfalls mit katalytisch aktiven Metallsalzen imprägniert sein können. Geeignete saure Ionentauscher auf Basis von Polystyrolsulfonsäuren, die als Festphasenkatalysatoren eingesetzt werden können, sind beispielsweise von der Firma Rohm & Haas unter der Markenbezeichnung Amberlyst^{®} erhältlich.

Die erfindungsgemäße Herstellung der Ester erfolgt durch Vermischen von Carbonsäure, Alkohol und Katalysator und nachfolgende Bestrahlung des Gemisches mit Mikrowellen in einem Reaktionsrohr, dessen Längsachse sich in der Ausbreitungsrichtung der Mikrowellen in einem Monomode-Mikrowellenapplikator befindet.

Die Bestrahlung des Reaktionsgemisches erfolgt mit Mikrowellen in einem weitgehend mikrowellentransparenten Reaktionsrohr, das sich innerhalb eines mit einem Mikrowellengenerator verbundenen Hohlleiters befindet. Bevorzugt fluchtet das Reaktionsrohr axial mit der zentralen Symmetrieachse des Hohlleiters.

Der als Mikrowellenapplikator fungierende Hohlleiter ist bevorzugt als Hohlraumresonator ausgeformt. Weiterhin bevorzugt werden die im Hohlleiter nicht absorbierten Mikrowellen an seinem Ende reflektiert. Bevorzugt wird die Länge des Hohlraumresonators so dimensioniert, dass sich in ihm eine stehende Welle ausbildet. Durch Ausformung des Mikrowellenapplikators als Resonator vom Reflexionstyp werden eine lokale Erhöhung der elektrischen Feldstärke bei gleicher vom Generator zugeführter Leistung und eine erhöhte Energieausnutzung erzielt.

Der Hohlraumresonator wird bevorzugt im E₀₁ₙ-Mode betrieben, wobei n für eine ganze Zahl steht und die Anzahl der Feldmaxima der Mikrowelle entlang der zentralen Symmetrieachse des Resonators angibt. Bei diesem Betrieb ist das elektrische Feld in Richtung der zentralen Symmetrieachse des Hohlraumresonators gerichtet. Es hat im Bereich der zentralen Symmetrieachse ein Maximum und nimmt zur Mantelfläche hin auf den Wert null ab. Diese Feldkonfiguration liegt rotationssymmetrisch um die zentrale Symmetrieachse vor. Durch Verwendung eines Hohlraumresonators mit einer Länge, bei der n eine ganze Zahl ist, wird die Ausbildung einer stehenden Welle ermöglicht. Je nach der gewünschten Strömungsgeschwindigkeit des Reaktionsguts durch das Reaktionsrohr, der benötigten Temperatur und der benötigten Verweilzeit im Resonator wird die Länge des Resonators relativ zu der Wellenlänge der eingesetzten Mikrowellenstrahlung ausgewählt. Bevorzugt ist n eine ganze Zahl von 1 bis 200, besonders bevorzugt von 2 bis 100, insbesondere von 4 bis 50 speziell von 3 bis 20 wie beispielsweise drei, vier, fünf, sechs, sieben, acht, neun oder zehn.

Die E₀₁ₙ-Mode des Hohlraumresonators wird in Englischer Sprache auch als TM₀₁ₙ-Mode bezeichnet, siehe beispielsweise K. Lange, K.H. Löcherer, Taschenbuch der Hochfrequenztechnik", Band 2, Seite K21 ff.

Die Einstrahlung der Mikrowellenenergie in den als Mikrowellenapplikator fungierenden Hohlleiter kann über geeignet dimensionierte Löcher oder Schlitze erfolgen. In einer erfindungsgemäß besonders bevorzugten Ausführungsform erfolgt die Bestrahlung des Reaktionsgemischs mit Mikrowellen in einem Reaktionsrohr, das sich in einem Hohlleiter mit koaxialem Übergang der Mikrowellen befindet. Für dieses Verfahren besonders bevorzugte Mikrowelleneinrichtungen sind aus einem Hohlraumresonator, einer Koppeleinrichtung zum Einkoppeln eines Mikrowellenfeldes in den Hohlraumresonator und mit je einer Öffnung an zwei gegenüber liegenden Stirnwänden zum Hindurchführen des Reaktionsrohres durch den Resonator aufgebaut. Die Einkopplung der Mikrowellen in den Hohlraumresonator erfolgt bevorzugt über einen Koppelstift, der in den Hohlraumresonator hineinragt. Bevorzugt ist der Koppelstift als ein als Kopplungsantenne fungierendes, bevorzugt metallisches Innenleiterrohr ausgeformt. In einer besonders bevorzugten Ausführungsform ragt dieser Koppelstift durch eine der stirnseitigen Öffnungen in den Hohlraumresonator hinein. Besonders bevorzugt schließt sich das Reaktionsrohr an das Innenleiterrohr des koaxialen Übergangs an und speziell wird es durch dessen Hohlraum hindurch in den Hohlraumresonator geführt. Bevorzugt fluchtet das Reaktionsrohr axial mit einer zentralen Symmetrieachse des Hohlraumresonators. Dazu weist der Hohlraumresonator bevorzugt je eine zentrische Öffnung an zwei gegenüber liegenden Stirnwänden zum Hindurchführen des Reaktionsrohres auf.

Die Einspeisung der Mikrowellen in den Koppelstift bzw. in das als Kopplungsantenne fungierende Innenleiterrohr kann beispielsweise mittels einer koaxialen Anschlussleitung erfolgen. In einer bevorzugten Ausführungsform wird das Mikrowellenfeld über einen Hohlleiter dem Resonator zugeführt, wobei das aus dem Hohlraumresonator herausragende Ende des Koppelstifts in eine Öffnung, die sich in der Wand des Hohlleiters befindet, in den Hohlleiter hineingeführt ist und von dem Hohlleiter Mikrowellenenergie entnimmt und in den Resonator koppelt.

In einer speziellen Ausführungsform erfolgt die Bestrahlung des Reaktionsgemisches mit Mikrowellen in einem mikrowellentransparenten Reaktionsrohr, das sich axialsymmetrisch in einem E₀₁ₙ-Rundhohlielter mit koaxialem Übergang der Mikrowellen befindet. Dabei wird das Reaktionsrohr durch den Hohlraum eines als Kopplungsantenne fungierenden Innenleiterrohres in den Hohlraumresonator geführt. In einer weiteren bevorzugten Ausführungsform erfolgt die Bestrahlung des Reaktionsgemischs mit Mikrowellen in einem mikrowellentransparenten Reaktionsrohr, das durch einen E₀₁ₙ-Hohlraumresonator mit axialer Einspeisung der Mikrowellen geführt wird, wobei die Länge des Hohlraumresonators so bemessen ist, dass sich n = 2 oder mehr Feldmaxima der Mikrowelle ausbilden.. In einer weiteren bevorzugten Ausführungsform erfolgt die Bestrahlung des Reaktionsgemisches mit Mikrowellen in einem mikrowellentransparenten Reaktionsrohr, das durch einen E₀₁ₙ-Hohlraumresonator mit axialer Einspeisung der Mikrowellen geführt wird, wobei die Länge des Hohlraumresonators so bemessen ist, dass sich eine stehende Welle mit n = 2 oder mehr Feldmaxima der Mikrowelle ausbildet. In einer weiteren bevorzugten Ausführungsform erfolgt die Bestrahlung des Reaktionsgemisches mit Mikrowellen in einem mikrowellentransparenten Reaktionsrohr, das sich axialsymmetrisch in einem kreiszylindrischen E₀₁ₙ-Hohlraumresonator mit koaxialem Übergang der Mikrowellen befindet, wobei die Länge des Hohlraumresonators so bemessen ist, dass sich n = 2 oder mehr Feldmaxima der Mikrowelle ausbilden. In einer weiteren bevorzugten Ausführungsform erfolgt die Bestrahlung des Reaktionsgemisches mit Mikrowellen in einem mikrowellentransparenten Reaktionsrohr, das sich axialsymmetrisch in einem kreiszylindrischen E₀₁ₙ-Hohlraumresonator mit koaxialem Übergang der Mikrowellen befindet, wobei die Länge des Hohlraumresonators so bemessen ist, dass sich eine stehende Welle mit n = 2 oder mehr Feldmaxima der Mikrowelle ausbildet.

Mikrowellengeneratoren, wie beispielsweise das Magnetron, das Klystron und das Gyrotron sind dem Fachmann bekannt.

Die zur Durchführung des erfindungsgemäßen Verfahrens eingesetzten Reaktionsrohre sind bevorzugt aus weitgehend mikrowellentransparentem, hoch schmelzendem Material gefertigt. Besonders bevorzugt werden nichtmetallische Reaktionsrohre eingesetzt. Unter weitgehend mikrowellentransparent werden hier Werkstoffe verstanden, die möglichst wenig Mikrowellenenergie absorbieren und in Wärme umwandeln. Als Maß für die Fähigkeit eines Stoffes, Mikrowellenenergie zu absorbieren und in Wärme zu überführen wird oftmals der dielektrische Verlustfaktor tan δ = ε"/ε' herangezogen. Der dielektrische Verlustfaktor tan δ ist definiert als das Verhältnis aus dielektrischem Verlust ε" und Dielektrizitätskonstante ε'. Beispiele für tan δ-Werte verschiedener Materialien sind beispielsweise in D. Bogdal, Microwave-assisted Organic Synthesis, Elsevier 2005 wiedergegeben. Für erfindungsgemäß geeignete Reaktionsrohre werden Materialen mit bei 2,45 GHz und 25 °C gemessenen tan δ-Werten von unter 0,01, insbesondere unter 0,005 und speziell unter 0,001 bevorzugt. Als bevorzugte mikrowellentransparente und temperaturstabile Materialien kommen in erster Linie Werkstoffe auf mineralischer Basis wie beispielsweise Quarz, Aluminiumoxid, Saphir, Zirkonoxid, Siliziumnitrid und ähnliches in Betracht. Auch temperaturstabile Kunststoffe wie insbesondere Fluorpolymere wie beispielsweise Teflon, und technische Kunststoffe wie Polypropylen, oder Polyaryletherketone wie beispielsweise glasfaserverstärktes Polyetheretherketon (PEEK) sind als Rohrmaterialien geeignet. Um den Temperaturbedingungen während der Reaktion zu widerstehen haben sich insbesondere mit diesen Kunststoffen beschichtete Mineralien wie Quarz oder Aluminiumoxid als Reaktormaterialien bewährt.

Für das erfindungsgemäße Verfahren besonders geeignete Reaktionsrohre haben einen Innendurchmesser von einem Millimeter bis 50 cm, insbesondere zwischen 2 mm und 35 cm und speziell zwischen 5 mm und 15 cm wie beispielsweise zwischen 10 mm und 7 cm. Unter Reaktionsrohren werden hier Gefäße verstanden, deren Verhältnis von Länge zu Durchmesser größer als 5, bevorzugt zwischen 10 und 100.000, besonders bevorzugt zwischen 20 und 10.000 wie beispielsweise zwischen 30 und 1.000 ist. Unter der Länge des Reaktionsrohres wird hier die Strecke des Reaktionsrohres verstanden, auf der die Mikrowellenbestrahlung erfolgt. In das Reaktionsrohr können Stromstörer und/oder andere Mischelemente eingebaut sein.

Für das erfindungsgemäße Verfahren besonders geeignete E₀₁-Hohlraumresonatoren haben bevorzugt einen Durchmesser, der mindestens der halben Wellenlänge der verwendeten Mikrowellenstrahlung entspricht. Bevorzugt beträgt der Durchmesser des Hohlraumresonators das 1,0- bis 10-fache, besonders bevorzugt das 1,1- bis 5-fache und insbesondere das 2,1- bis 2,6-fache der halben Wellenlänge der verwendeten Mikrowellenstrahlung.

Bevorzugt hat der E₀₁-Hohlraumresonator einen runden Querschnitt, was auch als E₀₁-Rundhohlleiter bezeichnet wird. Besonders bevorzugt hat er eine zylindrische Form und speziell eine kreiszylindrische Form.

Das Reaktionsrohr ist üblicherweise am Einlass mit einer Dosierpumpe sowie einem Manometer und am Auslass mit einer Druckhaltevorrichtung und einem Wärmetauscher versehen. Damit sind Reaktionen in einem sehr weiten Druck- und Temperaturbereich möglich.

Die Herstellung des Reaktionsgemischs aus Carbonsäure, Alkohol und Katalysator kann kontinuierlich, diskontinuierlich oder auch in semi-Batch-Prozessen durchgeführt werden. So kann die Herstellung des Reaktionsgemischs in einem vorgelagerten (semi)-Batch Prozess durchgeführt werden wie beispielsweise in einem Rührbehälter. In einer bevorzugten Ausführungsform werden die Edukte Carbonsäure und Alkohol sowie der Katalysator, unabhängig voneinander gegebenenfalls mit Lösemittel verdünnt, erst kurz vor dem Eintritt in das Reaktionsrohr vermischt. Der Katalysator kann als solches oder als Mischung mit einem der Edukte dem Reaktionsgemisch zugefügt werden. So hat es sich besonders bewährt, die Mischung aus Carbonsäure, Alkohol und Katalysator in einer Mischstrecke vorzunehmen, aus der das Reaktionsgemisch in das Reaktionsrohr gefördert wird. Weiterhin bevorzugt werden die Edukte und Katalysator dem erfindungsgemäßen Verfahren in flüssiger Form zugeführt. Dazu können höher schmelzende und/oder höher viskose Edukte beispielsweise in geschmolzenem Zustand und/oder mit Lösemittel versetzt beispielsweise als Lösung, Dispersion oder Emulsion eingesetzt werden. Der Katalysator wird einem der Edukte oder auch der Eduktmischung vor dem Eintritt in das Reaktionsrohr zugesetzt. Auch heterogene Systeme können nach dem erfindungsgemäßen Verfahren umgesetzt werden, wobei entsprechende technische Vorrichtungen zum Fördern des Reaktionsgutes erforderlich sind.

Das Reaktionsgemisch kann in das Reaktionsrohr entweder an dem durch das Innenleiterrohr geführte Ende eingespeist werden als auch an dem entgegen gesetzten Ende. Das Reaktionsgemisch kann folglich parallel oder antiparallel zur Ausbreitungsrichtung der Mikrowellen durch den Mikrowellenapplikator geführt werden.

Durch Variation von Rohrquerschnitt, Länge der Bestrahlungszone (hierunter wird die Strecke des Reaktionsrohres verstanden, in dem das Reaktionsgut Mikrowellenstrahlung ausgesetzt ist), Fließgeschwindigkeit, Geometrie des Hohlraumresonators sowie der eingestrahlten Mikrowellenleistung werden die Reaktionsbedingungen bevorzugt so eingestellt, dass die maximale Reaktionstemperatur schnellstmöglich erreicht wird und die Verweilzeit bei Maximaltemperatur so kurz bleibt, dass so wenig Neben- oder Folgereaktionen wie möglich auftreten. Das Reaktionsgut kann zur Vervollständigung der Reaktion, gegebenenfalls nach Zwischenkühlung, mehrfach das Reaktionsrohr durchlaufen. Bei langsamer verlaufenden Reaktionen hat es sich oftmals bewährt, das Reaktionsprodukt nach Verlassen des Reaktionsrohres noch eine gewisse Zeit bei Reaktionstemperatur zu halten. Vielfach hat sich bewährt, wenn das Reaktionsprodukt unmittelbar nach Verlassen des Reaktionsrohres z. B. durch Mantelkühlung oder Entspannung abgekühlt wird. Auch hat es sich bewährt, den Katalysator unmittelbar nach Verlassen des Reaktionsrohres zu deaktivieren. Dies kann beispielsweise durch Neutralisation oder bei heterogen katalysierten Reaktionen durch Filtration geschehen.

Bevorzugt wird der durch die Mikrowellenbestrahlung bedingte Temperaturanstieg beispielsweise durch Regelung der Mikrowellenintensität, der Durchflussgeschwindigkeit und/oder durch Kühlung des Reaktionsrohres, beispielsweise durch einen Stickstoffstrom, auf maximal 500 °C begrenzt. Besonders bewährt hat sich die Durchführung der Umsetzung bei Temperaturen zwischen 120 °C und maximal 400 °C und speziell zwischen 150 °C und maximal 300 °C wie beispielsweise bei Temperaturen zwischen 180 °C und 270 °C.

Die Dauer der Mikrowellenbestrahlung hängt von verschiedenen Faktoren wie beispielsweise der Geometrie des Reaktionsrohres, der eingestrahlten Mikrowellenenergie, der speziellen Reaktion und dem gewünschten Umsetzungsgrad ab. Üblicherweise wird die Mikrowellenbestrahlung über einen Zeitraum von weniger als 30 Minuten, bevorzugt zwischen 0,01 Sekunden und 15 Minuten, besonders bevorzugt zwischen 0,1 Sekunden und 10 Minuten und insbesondere zwischen einer Sekunde und 5 Minuten wie beispielsweise zwischen 5 Sekunden und 2 Minuten vorgenommen. Die Intensität (Leistung) der Mikrowellenstrahlung wird dabei so eingestellt, dass das Reaktionsgut beim Verlassen des Hohlraumresonators die gewünschte Maximaltemperatur hat. In einer bevorzugten Ausführungsform wird das Umsetzungsprodukt direkt nach Beendigung der Mikrowellenbestrahlung möglichst schnell auf Temperaturen unterhalb 120 °C, bevorzugt unterhalb 100 °C und speziell unterhalb 60 °C abgekühlt.

Bevorzugt wird die Umsetzung bei Drücken zwischen 1 bar (Atmosphärendruck) und 500 bar, besonders bevorzugt zwischen 1,5 und 200 bar, insbesondere zwischen 3 bar und 150 bar und speziell zwischen 10 bar und 100 bar wie beispielsweise zwischen 15 und 50 bar durchgeführt. Besonders bewährt hat sich das Arbeiten unter erhöhtem Druck, wobei oberhalb der Siedetemperatur (bei Normaldruck) der Edukte, Produkte, des gegebenenfalls anwesenden Lösemittels und/oder des während der Reaktion gebildeten Reaktionswassers gearbeitet wird. Besonders bevorzugt wird der Druck so hoch eingestellt, dass das Reaktionsgemisch während der Mikrowellenbestrahlung im flüssigen Zustand verbleibt und nicht siedet.

Zur Vermeidung von Nebenreaktionen und zur Herstellung von möglichst reinen Produkten hat es sich bewährt, Edukte und Produkte in Gegenwart eines inerten Schutzgases wie beispielsweise Stickstoff, Argon oder Helium zu handhaben.

Auch wenn die Edukte Carbonsäure und Alkohol oftmals zu gut handhabbaren Reaktionsgemischen führen hat es sich in vielen Fällen bewährt, in Gegenwart von Lösemitteln zu arbeiten um beispielsweise die Viskosität des Reaktionsmediums abzusenken und/oder das Reaktionsgemisch, insbesondere sofern es heterogen ist, zu fluidisieren. Dafür können prinzipiell alle Lösemittel eingesetzt werden, die unter den angewendeten Reaktionsbedingungen inert sind und nicht mit den Edukten bzw. den gebildeten Produkten reagieren. Ein wichtiger Faktor bei der Auswahl geeigneter Lösemittel ist deren Polarität, die einerseits die Löseeigenschaften und andererseits das Ausmaß der Wechselwirkung mit Mikrowellenstrahlung bestimmt. Ein besonders wichtiger Faktor bei der Auswahl geeigneter Lösemittel ist deren dielektrischer Verlust ε". Der dielektrische Verlust ε" beschreibt den Anteil an Mikrowellenstrahlung, der bei der Wechselwirkung einer Substanz mit Mikrowellenstrahlung in Wärme überführt wird. Letzt genannter Wert hat sich als besonders wichtiges Kriterium für die Eignung eines Lösemittels für die Durchführung des erfindungsgemäßen Verfahrens erwiesen.

Besonders bewährt hat sich das Arbeiten in Lösemitteln, die eine möglichst geringe Mikrowellenabsorption zeigen und somit nur einen kleinen Beitrag zur Erwärmung des Reaktionssystems liefern. Für das erfindungsgemäße Verfahren bevorzugte Lösemittel besitzen einen bei Raumtemperatur und 2450 MHz gemessenen dielektrischen Verlust ε" von weniger als 10 und vorzugsweise weniger als 1 wie beispielsweise weniger als 0,5. Eine Übersicht über den dielektrischen Verlust verschiedener Lösemittel findet sich zum Beispiel in "Microwave Synthesis" von B. L. Hayes, CEM Publishing 2002. Für das erfindungsgemäße Verfahren geeignet sind insbesondere Lösemittel mit ε"-Werten unterhalb 10 wie N-Methylpyrrolidon, N,N-Dimethylformamid oder Aceton, und insbesondere Lösemittel mit ε"-Werten unterhalb 1. Beispiele für besonders bevorzugte Lösemittel mit ε"-Werten unterhalb 1 sind aromatische und/oder aliphatische Kohlenwasserstoffe wie beispielsweise Toluol, Xylol, Ethylbenzol, Tetralin, Hexan, Cyclohexan, Decan, Pentadecan, Dekalin sowie kommerzielle Kohlenwasserstoffgemische wie Benzinfraktionen, Kerosin, Solvent Naphtha, ^{®}Shellsol AB, Solvesso^{®} 150, Solvesso^{®} 200, Exxsol^{®}, Isopar^{®} und Shellsol^{®}-Typen. Lösemittelgemische, die ε"-Werte bevorzugt unterhalb 10 und speziell unterhalb 1 aufweisen, sind für die Durchführung des erfindungsgemäßen Verfahrens gleichermaßen bevorzugt.

In einer weiteren bevorzugten Ausführungsform wird das erfindungsgemäße Verfahren in Lösemitteln mit höheren ε"-Werten von beispielsweise 5 und höher wie insbesondere mit ε"-Werten von 10 und höher durchgeführt. Diese Ausführungsform hat sich insbesondere bei der Umsetzung von Reaktionsgemischen bewährt, die selbst, das heißt ohne Anwesenheit von Löse- und/oder Verdünnungsmitteln nur eine sehr geringe Mikrowellenabsorption zeigen. So hat sich diese Ausführungsform insbesondere bei Reaktionsgemischen bewährt, die einen dielektrischen Verlust ε" von weniger als 10 und vorzugsweise weniger als 1 aufweisen. Die durch die Lösemittelzugabe oftmals beobachtete beschleunigte Aufheizung des Reaktionsgemischs erfordert jedoch Maßnahmen zur Einhaltung der Maximaltemperatur.

Sofern in Gegenwart von Lösemitteln gearbeitet wird, liegt deren Anteil an der Reaktionsmischung bevorzugt zwischen 1 und 95 Gew.-%, besonders bevorzugt zwischen 2 und 90 Gew.-%, speziell zwischen 5 und 85 Gew.-% und insbesondere zwischen 10 und 75 Gew.-% wie beispielsweise zwischen 30 und 60 Gew.-%. Besonders bevorzugt wird die Reaktion lösemittelfrei durchgeführt.

In einer weiteren bevorzugten Ausführungsform werden dem Reaktionsgemisch in diesem unlösliche, stark Mikrowellen absorbierende Substanzen zugegeben. Diese führen zu einer starken lokalen Erhitzung des Reaktionsgemischs und in der Folge zu weiter beschleunigten Reaktionen. Ein geeigneter derartiger Wärmesammler ist beispielsweise Graphit.

Als Mikrowellen werden elektromagnetische Strahlen mit einer Wellenlänge zwischen etwa 1 cm und 1m und Frequenzen zwischen etwa 300 MHz und 30 GHz bezeichnet. Dieser Frequenzbereich ist prinzipiell für das erfindungsgemäße Verfahren geeignet. Bevorzugt wird für das erfindungsgemäße Verfahren Mikrowellenstrahlung mit den für industrielle, wissenschaftliche, medizinische, häusliche oder ähnliche Anwendungen freigegebenen Frequenzen verwendet wie beispielsweise mit Frequenzen von 915 MHz, 2,45 GHz, 5,8 GHz oder 24,12 GHz.

Die für die Durchführung des erfindungsgemäßen Verfahrens in den Hohlraumresonator einzustrahlende Mikrowellenleistung ist insbesondere abhängig von der angestrebten Reaktionstemperatur, aber auch von der Geometrie des Reaktionsrohrs und damit des Reaktionsvolumens sowie der Durchflussgeschwindigkeit des Reaktionsguts durch die Heizzone. Sie liegt üblicherweise zwischen 200 W und mehreren 100 kW und insbesondere zwischen 500 W und 100 kW wie beispielsweise zwischen 1 kW und 70 kW. Sie kann über einen oder mehrere Mikrowellengeneratoren erzeugt werden.

In einer bevorzugten Ausführungsform wird die Reaktion in einem druckfesten, chemisch inerten Rohr durchgeführt, wobei das sich bildende Reaktionswasser sowie gegebenenfalls Edukte und, sofern anwesend, Lösemittel zu einem Druckaufbau führen. Nach Beendigung der Reaktion kann der Überdruck durch Entspannung zur Verflüchtigung und Abtrennung von Reaktionswasser, überschüssigen Edukten sowie gegebenenfalls Lösemittel und/oder zur Abkühlung des Reaktionsprodukts verwendet werden. In einer weiteren Ausführungsform wird das gebildete Reaktionswasser nach dem Abkühlen und/oder Entspannen durch übliche Verfahren wie beispielsweise Phasentrennung, Destillation, Strippen, Flashen und/oder Absorption abgetrennt.

Zur Erzielung besonders hoher Umsetzungsgrade hat es sich in vielen Fällen bewährt, das erhaltene Reaktionsprodukt nach Entfernen von Reaktionswasser sowie gegebenenfalls Austragen von Produkt und/oder Nebenprodukt erneut der Mikrowellenbestrahlung auszusetzen, wobei gegebenenfalls das Verhältnis der eingesetzten Reaktanden um verbrauchte oder unterschüssige Edukte zu ergänzen ist.

Die Vorteile des erfindungsgemäßen Verfahrens liegen in einer sehr gleichmäßigen Bestrahlung des Reaktionsgutes im Zentrum eines symmetrischen Mikrowellenfeldes innerhalb eines Reaktionsrohres, dessen Längsachse sich in der Ausbreitungsrichtung der Mikrowellen eines Monomode-Mikrowellenapplikators und insbesondere innerhalb eines E₀₁-Hohlraumresonators beispielsweise mit koaxialem Übergang befindet. Dabei erlaubt das erfindungsgemäße Reaktordesign die Durchführung von Reaktionen auch bei sehr hohen Drücken und/oder Temperaturen. Durch Temperatur- und/oder Druckerhöhung wird eine deutliche Steigerung von Umsetzungsgrad und Ausbeute auch gegenüber bekannten Mikrowellenreaktoren beobachtet, ohne dass es dabei zu unerwünschten Nebenreaktionen und/oder Verfärbungen kommt. Überraschenderweise wird dabei ein sehr hoher Wirkungsgrad bei der Ausnutzung der in den Hohlraumresonator eingestrahlten Mikrowellenenergie erzielt, der üblicherweise über 50 %, oftmals über 80 %, teilweise über 90 % und in speziellen Fällen über 95 % wie beispielsweise über 98 % der eingestrahlten Mikrowellenleistung liegt und somit ökonomische wie auch ökologische Vorteile gegenüber konventionellen Herstellverfahren wie auch gegenüber Mikrowellenverfahren des Standes der Technik bietet.

Das erfindungsgemäße Verfahren erlaubt zudem eine kontrollierte, sichere und reproduzierbare Reaktionsführung. Da das Reaktionsgut im Reaktionsrohr parallel zur Ausbreitungsrichtung der Mikrowellen bewegt wird, werden bekannte Überhitzungsphänomene durch unkontrollierbare Feldverteilungen, die zu lokalen Überhitzungen durch wechselnde Intensitäten des Mikrowellenfeldes beispielsweise in Wellenbergen und Knotenpunkten führen, durch die Fließbewegung des Reaktionsgutes ausgeglichen. Die genannten Vorteile erlauben es auch, mit hohen Mikrowellenleistungen von mehr als 1 kW wie beispielsweise 2 bis 10 kW und insbesondere 5 bis 100 kW und teilweise auch höher zu arbeiten und somit in Kombination mit einer nur kurzen Verweilzeit im Hohlraumresonator große Produktionsmengen von 100 und mehr Tonnen pro Jahr in einer Anlage zu bewerkstelligen.

Dabei war es überraschend, dass trotz der im kontinuierlich durchflossenen Strömungsrohr nur sehr kurzen Verweildauer des Reaktionsgemischs im Mikrowellenfeld eine sehr weitgehende Veresterung mit Umsätzen im Allgemeinen von über 80 %, oftmals auch über 90 % wie beispielsweise über 95 % bezogen auf die im Unterschuss eingesetzte Komponente stattfindet, ohne Bildung nennenswerter Mengen an Nebenprodukten. Weiterhin überraschend war, dass sich die genannten Umsätze unter diesen Reaktionsbedingungen ohne Abtrennung des bei der Veresterung gebildeten Reaktionswassers erzielen lassen. Bei einer entsprechenden Umsetzung dieser Reaktionsgemische in einem Strömungsrohr gleicher Dimensionierung unter thermischer Mantelheizung werden zur Erzielung geeigneter Reaktionstemperaturen extrem hohe Wandtemperaturen benötigt, die zur Bildung undefinierter Polymere und gefärbter Spezies führten, aber bei gleichem Zeitintervall deutlich geringere Esterbildung bewirken. Des Weiteren besitzen die nach dem erfindungsgemäßen Verfahren hergestellten Produkte sehr niedrige Metallgehalte ohne dass es einer weiteren Aufarbeitung der Rohprodukte bedarf. So liegen die Metallgehalte der nach dem erfindungsgemäßen Verfahren hergestellten Produkte bezogen auf Eisen als Hauptelement üblicherweise unterhalb 25 ppm bevorzugt unterhalb 15 ppm, speziell unterhalb 10 ppm wie beispielsweise zwischen 0,01 und 5 ppm Eisen.

Das erfindungsgemäße Verfahren erlaubt somit eine sehr schnelle, energiesparende und kostengünstige Herstellung von Carbonsäureestern in hohen Ausbeuten und mit hoher Reinheit in großtechnischen Mengen. Bei diesem Verfahren fallen - neben Reaktionswasser - keine wesentlichen Mengen an Nebenprodukten an. Derartig schnelle und selektive Umsetzungen sind nach klassischen Methoden nicht zu erzielen und waren alleine durch Heizen auf hohe Temperaturen nicht zu erwarten.

### Beispiele

Die Umsetzungen der Reaktionsgemische unter Mikrowellenbestrahlung erfolgten in einem Keramikrohr (60 x 1 cm), das sich axialsymmetrisch in einem zylindrischen Hohlraumresonator (60 x 10 cm) befand. An einer der Stirnseiten des Hohlraumresonators verlief das Keramikrohr durch den Hohlraum eines als Kopplungsantenne fungierenden Innenleiterrohres. Das von einem Magnetron erzeugte Mikrowellenfeld mit einer Frequenz von 2,45 GHz wurde mittels der Kopplungsantenne in den Hohlraumresonator eingekoppelt (E₀₁-Hohlraumapplikator; Monomode), in dem sich eine stehende Welle ausbildete.

Die Mikrowellenleistung wurde über die Versuchsdauer jeweils in der Art eingestellt, dass die gewünschte Temperatur des Reaktionsguts am Ende der Bestrahlungszone konstant gehalten wurde. Die in den Versuchsbeschreibungen genannten Mikrowellenleistungen repräsentieren daher den zeitlichen Mittelwert der eingestrahlten Mikrowellenleistung. Die Temperaturmessung des Reaktionsgemischs wurde direkt nach Verlassen der Reaktionszone (etwa 15 cm Strecke in einer isolierten Edelstahlkapillare, Ø 1 cm) mittels Pt100 Temperatursensor vorgenommen. Vom Reaktionsgemisch nicht direkt absorbierte Mikrowellenenergie wurde an der der Kopplungsantenne entgegen liegenden Stirnseite des Hohlraumresonators reflektiert; die vom Reaktionsgemisch auch beim Rücklauf nicht absorbierte und in Richtung des Magnetrons zurück gespiegelte Mikrowellenenergie wurde mit Hilfe eines Prismensystems (Zirkulator) in ein Wasser enthaltendes Gefäß geleitet. Aus der Differenz zwischen eingestrahlter Energie und Aufheizung dieser Wasserlast wurde die in das Reaktionsgut eingetragene Mikrowellenenergie berechnet.

Mittels einer Hochdruckpumpe und eines geeigneten Druckentlastungsventils wurde die Reaktionsmischung im Reaktionsrohr unter einen solchen Arbeitsdruck gesetzt, der ausreichte, um alle Edukte und Produkte bzw. Kondensationsprodukte stets im flüssigen Zustand zu halten. Die aus Carbonsäure und Alkohol hergestellten Reaktionsmischungen wurden mit einer konstanten Flussrate durch das Reaktionsrohr gepumpt und die Verweilzeit in der Bestrahlungszone durch Modifizierung der Strömungsgeschwindigkeit eingestellt.

Die Analytik der Produkte erfolgte mittels ¹H-NMR-Spektroskopie bei 500 MHz in CDCl₃. Die Bestimmung von Eisengehalten erfolgte mittels Atomabsorptionsspektroskopie.

### Beispiel 1: Herstellung von Benzoesäuremethylester

In einem 10 l Büchi-Rührautoklaven mit Rührer, Innenthermometer und Druckausgleich wurden 2,36 kg Methanol (73,5 mol) vorgelegt und mit 3 kg Benzoesäure (24,5mol) sowie 50 g Methansulfonsäure versetzt.

Das so erhaltene Gemisch wurde bei einem Arbeitsdruck von 35 bar kontinuierlich mit 5 l/h durch das Reaktionsrohr gepumpt und einer Mikrowellenleistung von 2,0 kW ausgesetzt, von denen 96 % vom Reaktionsgut absorbiert wurden. Die Verweilzeit des Reaktionsgemischs in der Bestrahlungszone betrug ca. 34 Sekunden. Am Ende des Reaktionsrohres hatte das Reaktionsgemisch eine Temperatur von 260 °C. Das Reaktionsgemisch wurde direkt nach Verlassen des Reaktors mit einem Intensivwärmetauscher auf Raumtemperatur abgekühlt.

Es wurde ein Umsatz von 85 % d. Th. erreicht. Das Reaktionsprodukt war praktisch farblos und enthielt <1 ppm Eisen. Nach Neutralisation des Katalysators mit Hydrogencarbonat-Lösung und destillativer Abtrennung von Wasser und überschüssigem Methanol wurden mittels Vakuumdestillation 2,71 kg Benzoesäuremethylester mit einer Reinheit von >98 % erhalten.

### Beispiel 2: Herstellung von p-Tolylsäurebutylester

In einem 10 l Büchi-Rührautoklaven mit Rührer, Innenthermometer und Druckausgleich wurden 3,12 kg n-Butanol (42 mol) vorgelegt und mit 2,88 kg p-Tolylsäure (21 mol) sowie 60 g Methansulfonsäure versetzt.

Das so erhaltene Gemisch wurde bei einem Arbeitsdruck von 25 bar kontinuierlich mit 3 l/h durch das Reaktionsrohr gepumpt und einer Mikrowellenleistung von 3,2 kW ausgesetzt, von denen 92 % vom Reaktionsgut absorbiert wurden. Die Verweilzeit des Reaktionsgemischs in der Bestrahlungszone betrug ca. 57 Sekunden. Am Ende des Reaktionsrohres hatte das Reaktionsgemisch eine Temperatur von 272 °C. Das Reaktionsgemisch wurde direkt nach Verlassen des Reaktors mit einem Intensivwärmetauscher auf Raumtemperatur abgekühlt.

Es wurde ein Umsatz von 82 % d. Th. erreicht. Das Reaktionsprodukt war praktisch farblos und enthielt < 5 ppm Eisen. Nach Neutralisation des Katalysators mit Hydrogencarbonat-Lösung und destillativer Abtrennung von Wasser und überschüssigem Butanol wurden mittels Vakuumdestillation 3,3 kg Tolylsäurebutylester mit einer Reinheit von >97,5 % erhalten.

### Beispiel 3: Herstellung von p-Hydroxybenzoesäuremethylester

In einem 10 l Büchi-Rührautoklaven mit Rührer, Innenthermometer und Druckausgleich wurden 2,7 kg Methanol (84 mol) vorgelegt und darin unter Erwärmen auf 60 °C mit 3,8 kg p-Hydroxybenzoesäure (27,5 mol) sowie 65 g Schwefelsäure (96 %ig) gelöst.

Die so erhaltene homogene Lösung wurde bei einem Arbeitsdruck von 38 bar kontinuierlich mit 6,2 l/h durch das Reaktionsrohr gepumpt und einer Mikrowellenleistung von 2,8 kW ausgesetzt, von denen 94 % vom Reaktionsgut absorbiert wurden. Die Verweilzeit des Reaktionsgemischs in der Bestrahlungszone betrug ca. 27 Sekunden. Am Ende des Reaktionsrohres hatte das Reaktionsgemisch eine Temperatur von 267 °C. Das Reaktionsgemisch wurde direkt nach Verlassen des Reaktors mit einem Intensivwärmetauscher auf Raumtemperatur abgekühlt.

Es wurde ein Umsatz von 87 % d. Th. erreicht. Das Reaktionsprodukt war praktisch farblos und enthielt < 2 ppm Eisen. Nach Neutralisation des Katalysators mit Ammoniumhydrogencarbont-Lösung und destillativer Abtrennung von Wasser und überschüssigem Methanol wurden 3,6 kg roher p-Hydroxybenzoesäuremethylester in Mischung mit Ammoniumsulfat und unumgesetztem Edukt erhalten. Durch Rekristallisation aus Methanol wurde das Produkt mit einer Reinheit von > 99 % isoliert.

### Beispiel 4: Herstellung von oligomerem PET

In einem 10 l Büchi-Rührautoklaven mit Rührer, Innenthermometer und Druckausgleich wurden 1,86 kg Ethylenglykol (30 mol) vorgelegt, auf 120 °C erwärmt und darin 4,14 kg Terephthalsäure (25 mol) gelöst.

Die so erhaltene homogene Lösung wurde bei einem Arbeitsdruck von 30 bar kontinuierlich mit 6 l/h durch das Reaktionsrohr gepumpt und einer Mikrowellenleistung von 3 kW ausgesetzt, von denen 92 % vom Reaktionsgut absorbiert wurden. Die Verweilzeit des Reaktionsgemischs in der Bestrahlungszone betrug ca. 28 Sekunden. Am Ende des Reaktionsrohres hatte das Reaktionsgemisch eine Temperatur von 255 °C. Das Reaktionsgemisch wurde direkt nach Verlassen des Reaktors mit einem Intensivwärmetauscher auf Raumtemperatur abgekühlt.

Das Reaktionsprodukt war deutlich viskos und praktisch farblos. Es enthielt < 5 ppm Eisen.

Vor der Reaktion wurde eine Säurezahl von 471 (mg KOH/g Probe) mittels Titration bestimmt. Nach der Reaktion betrug der Wert 52. Demzufolge betrug der Umsatz bezogen auf die eingesetzten COOH Funktionalitäten etwa 89 %. Gemäß Statistik der Polykondensation betrug somit die mittlere Kettenlänge der Oligomere 8 -10 PET-Einheiten oder ungefähr 1700 g/mol.

### Beispiel 5: Herstellung von 2,6-Pyridindicarbonsäuredimethylester

In einem 10 l Büchi-Rührautoklaven mit Rührer, Innenthermometer und Druckausgleich wurden 3,9 kg Methanol (120 mol) vorgelegt und mit 2 kg 2,6-Pyridindicarbonsäure (12 mol) sowie 60 g Methansulfonsäure versetzt und durch leichtes Erwärmen auf 60 °C ineinander gelöst.

Das so erhaltene Gemisch wurde bei einem Arbeitsdruck von 35 bar kontinuierlich mit 4 l/h durch das Reaktionsrohr gepumpt und einer Mikrowellenleistung von 2,4 kW ausgesetzt, von denen 93 % vom Reaktionsgut absorbiert wurden. Die Verweilzeit des Reaktionsgemischs in der Bestrahlungszone betrug ca. 42 Sekunden. Am Ende des Reaktionsrohres hatte das Reaktionsgemisch eine Temperatur von 240 °C. Das Reaktionsgemisch wurde direkt nach Verlassen des Reaktors mit einem Intensivwärmetauscher auf Raumtemperatur abgekühlt.

Es wurde ein Umsatz von 83 % d. Th. erreicht. Das Reaktionsprodukt war praktisch farblos und enthielt < 5 ppm Eisen. Nach Neutralisation des Katalysators mit Hydrogencarbonat-Lösung und destillativer Abtrennung von Wasser und überschüssigem Methanol wurden destillativ 1,8 kg 2,6-Pyridindicarbonsäuredimethylester mit einer Reinheit von > 99 % erhalten.

## Patentansprüche

1. Kontinuierliches Verfahren zur Herstellung von aromatischen Carbonsäureestern, in dem mindestens eine aromatische Carbonsäure der Formel (I)
Ar-COOH (I)
worin Ar für einen gegebenenfalls substituierten Arylrest mit 5 bis 50 Atomen steht,
mit mindestens einem Alkohol der Formel (II)
R²-(OH)ₙ (II)
worin
R² für einen gegebenenfalls substituierten Kohlenwasserstoffrest mit 1 bis 100 C-Atomen und
n für eine Zahl von 1 bis 10 stehen,
in Gegenwart mindestens eines Veresterungskatalysators unter Mikrowellenbestrahlung in einem Reaktionsrohr, dessen Längsachse sich in der Ausbreitungsrichtung der Mikrowellen eines Monomode-Mikrowellenapplikators befindet, zum Ester umgesetzt wird, bei dem die Bestrahlung des Reaktionsgemischs mit Mikrowellen in einem weitgehend mikrowellentransparenten Reaktionsrohr innerhalb eines mit einem Mikrowellengenerator über Wellenleiter verbundenen Hohlleiters erfolgt.

2. Verfahren nach Anspruch 1, bei dem der Mikrowellenapplikator als Hohlraumresonator ausgestaltet ist.

3. Verfahren nach Anspruch 1 und/oder 2, bei dem der Mikrowellenapplikator als Hohlraumresonator vom Reflexionstyp ausgestaltet ist.

4. Verfahren nach einem oder mehreren der Ansprüche 1 bis 3, bei dem das Reaktionsrohr axial mit einer zentralen Symmetrieachse des Hohlleiters fluchtet.

5. Verfahren nach einem oder mehreren der Ansprüche 1 bis 4, bei dem die Bestrahlung des Reaktionsgemisches in einem Hohlraumresonator mit koaxialem Übergang der Mikrowellen erfolgt.

6. Verfahren nach einem oder mehreren der Ansprüche 1 bis 5, bei dem der Hohlraumresonator im E₀₁ₙ-Mode betrieben wird, wobei n eine ganze Zahl von 1 bis 200 ist.

7. Verfahren nach einem oder mehreren der Ansprüche 1 bis 6, bei dem sich im Hohlraumresonator eine stehende Welle ausbildet.

8. Verfahren nach einem oder mehreren der Ansprüche 1 bis 7, bei dem das Reaktionsgut durch die Mikrowellenbestrahlung auf Temperaturen zwischen 150 und 500 °C erhitzt wird.

9. Verfahren nach einem oder mehreren der Ansprüche 1 bis 8, bei dem die Mikrowellenbestrahlung bei Drücken oberhalb des Atmosphärendrucks erfolgt.

10. Verfahren nach einem oder mehreren der Ansprüche 1 bis 9, bei dem Ar ein zyklisches, durchkonjugiertes System mit (4n + 2) π-Elektronen ist, worin n gleich 1, 2, 3, 4 oder 5 ist.

11. Verfahren nach einem oder mehreren der Ansprüche 1 bis 10, bei dem Ar eine Alkylarylgruppe mit mindestens einem Alkylrest mit 1 bis 50 C-Atomen ist.

12. Verfahren nach einem oder mehreren der Ansprüche 1 bis 11, bei dem Ar zwei oder mehr Hydroxylgruppen und/oder Hydroxyalkylgruppen trägt.

13. Verfahren nach einem oder mehreren der Ansprüche 1 bis 10, bei dem Ar ausgewählt ist aus Benzoesäure, Phthalsäure, Isophthalsäure, Terephthalsäure, den Isomeren der Naphthalincarbonsäure, der Pyridincarbonsäure und der Naphthalindicarbonsäure, Trimellitsäure, Trimesinsäure, Pyromellitsäure und Mellitsäure, den Isomeren der Methoxybenzoesäure, Hydroxybenzoesäure, Hydroxymethylbenzoesäure, Hydroxymethoxybenzoesäure, Hydroxydimethoxybenzoesäure, Hydroxyisophthalsäure, Hydroxynaphthalincarbonsäure, Hydoxypyridincarbonsäure, Hydroxymethylpyridincarbonsäure, Hydroxychinolincarbonsäure, o-Tolylsäure, m-Tolylsäure, p-Tolylsäure, o-Ethylbenzoesäure, m-Ethylbenzoesäure, p-Ethylbenzoesäure, o-Propylbenzoesäure, m-Propylbenzoesäure, p-Propylbenzoesäure, 3,4-Dimethylbenzoesäure, und Thiophencarbonsäure.

14. Verfahren nach einem oder mehreren der Ansprüche 1 bis 13, bei dem R² für einen gegebenenfalls substituierten aliphatischen Rest mit 2 bis 24 Kohlenstoffatomen steht.

15. Verfahren nach einem oder mehreren der Ansprüche 1 bis 13, bei dem R² für eine gegebenenfalls substituierte C₆-C₁₂-Arylgruppe oder eine gegebenenfalls substituierte heteroaromatische Gruppe mit 5 bis 12 Ringgliedern steht.

16. Verfahren nach einem oder mehreren der Ansprüche 1 bis 15, bei dem n gleich eins, zwei, drei, vier fünf oder sechs ist.

17. Verfahren nach einem oder mehreren der Ansprüche 1 bis 13, bei dem R² für Reste der Formel (III) steht
-(R⁴-O)ₙ-R⁵ (III)
worin
R⁴ für eine Alkylengruppe mit 2 bis 18 C-Atomen oder Mischungen daraus,
R⁵ für Wasserstoff oder einen Kohlenwasserstoffrest mit 1 bis 24 C-Atomen oder eine Gruppe der Formel -R⁴-NR¹⁰R¹¹,
n für eine Zahl zwischen 1 und 500 und
R¹⁰, R¹¹ unabhängig voneinander für einen aliphatischen Rest mit 1 bis 24 C-Atomen, eine Arylgruppe- oder Heteroarylgruppe mit 5 bis 12 Ringgliedern, eine Poly(oxyalkylen)gruppe mit 1 bis 50 Poly(oxyalkylen)einheiten, wobei sich die Polyoxyalkyleneinheiten von Alkylenoxideinheiten mit 2 bis 6 C-Atomen ableiten, oder R¹⁰ und R¹¹ gemeinsam mit dem Stickstoffatom, an das sie gebunden sind, einen Ring mit 4, 5, 6 oder mehr Ringgliedern bilden, stehen.

18. Verfahren nach einem oder mehreren der Ansprüche 1 bis 17, bei dem aromatische Carbonsäure (I) und Alkohol (II) im molaren Verhältnis von 20:1 bis 1:20, jeweils bezogen auf die Molequivalente an Carboxyl- und Hydroxylgruppen, zur Reaktion gebracht werden.

19. Verfahren nach einem oder mehreren der Ansprüche 1 bis 18, welches in Gegenwart von homogenen Katalysatoren, heterogenen Katalysatoren oder deren Mischungen durchgeführt wird.

## Claims

1. A continuous method for producing aromatic carboxylic esters, in which at least one aromatic carboxylic acid of the formula (I)
Ar-COOH (I)
in which Ar is an optionally substituted aryl radical having 5 to 50 atoms,
is reacted with at least one alcohol of the formula (II)
R²-(OH)ₙ (II)
in which
R² is an optionally substituted hydrocarbon radical having 1 to 100 carbon atoms and
n is a number from 1 to 10,
in the presence of at least one esterification catalyst with microwave irradiation in a reaction tube, the longitudinal axis of which extends in the direction of propagation of the microwaves of a monomode microwave applicator, to give the ester, in which the irradiation of the reaction mixture takes place with microwaves in a largely microwave-transparent reaction tube within a hollow conductor connected via waveguides to a microwave generator.

2. The method as claimed in claim 1, in which the microwave applicator is designed as a cavity resonator.

3. The method as claimed in claim 1 and/or 2, in which the microwave applicator is configured as a cavity resonator of the reflection type.

4. The method as claimed in one or more of claims 1 to 3, in which the reaction tube is aligned axially with a central axis of symmetry of the hollow conductor.

5. The method as claimed in one or more of claims 1 to 4, in which the irradiation of the reaction mixture takes place in a cavity resonator with a coaxial transition of the microwaves.

6. The method as claimed in one or more of claims 1 to 5, in which the cavity resonator is operated in the E₀₁ₙ mode, where n is an integer from 1 to 200.

7. The method as claimed in one or more of claims 1 to 6, in which a stationary wave is formed in the cavity resonator.

8. The method as claimed in one or more of claims 1 to 7, in which the reaction material is heated by the microwave irradiation to temperatures between 150 and 500°C.

9. The method as claimed in one or more of claims 1 to 8, in which the microwave irradiation takes place at pressures above atmospheric pressure.

10. The method as claimed in one or more of claims 1 to 9, in which Ar is a cyclic, through-conjugated system with (4n + 2) π electrons, in which n is 1, 2, 3, 4 or 5.

11. The method as claimed in one or more of claims 1 to 10, in which Ar is an alkylaryl group with at least one alkyl radical having 1 to 50 carbon atoms.

12. The method as claimed in one or more of claims 1 to 11, in which Ar carries two or more hydroxyl groups and/or hydroxyalkyl groups.

13. The method as claimed in one or more of claims 1 to 10, in which Ar is selected from benzoic acid, phthalic acid, isophthalic acid, terephthalic acid, the isomers of naphthalenecarboxylic acid, of pyridinecarboxylic acid and of naphthalenedicarboxylic acid, trimellitic acid, trimesic acid, pyromellitic acid and mellitic acid, the isomers of methoxybenzoic acid, hydroxybenzoic acid, hydroxymethylbenzoic acid, hydroxymethoxybenzoic acid, hydroxydimethoxybenzoic acid, hydroxyisophthalic acid, hydroxynaphthalenecarboxylic acid, hydroxypyridinecarboxylic acid, hydroxymethylpyridinecarboxylic acid, hydroxyquinolinecarboxylic acid, o-toluic acid, m-toluic acid, p-toluic acid, o-ethylbenzoic acid, m-ethylbenzoic acid, p-ethylbenzoic acid, o-propylbenzoic acid, m-propylbenzoic acid, p-propylbenzoic acid, 3,4-dimethylbenzoic acid, and thiophenecarboxylic acid.

14. The method as claimed in one or more of claims 1 to 13, in which R² is an optionally substituted aliphatic radical having 2 to 24 carbon atoms.

15. The method as claimed in one or more of claims 1 to 13, in which R² is an optionally substituted C₆-C₁₂-aryl group or an optionally substituted heteroaromatic group having 5 to 12 ring members.

16. The method as claimed in one or more of claims 1 to 15, in which n is one, two, three, four, five or six.

17. The method as claimed in one or more of claims 1 to 13, in which R² is radicals of the formula (III)
-(R⁴-O)ₙ-R⁵ (III)
in which
R⁴ is an alkylene group having 2 to 18 carbon atoms or mixtures thereof,
R⁵ is hydrogen or a hydrocarbon radical having 1 to 24 carbon atoms or a group of the formula -R⁴-NR¹⁰R¹¹,
n is a number between 1 and 500 and
R¹⁰, R¹¹ independently of one another, are an aliphatic radical having 1 to 24 carbon atoms, an aryl group or heteroaryl group having 5 to 12 ring members, a poly(oxyalkylene) group having 1 to 50 poly(oxyalkylene) units, where the polyoxyalkylene units are derived from alkylene oxide units having 2 to 6 carbon atoms, or R¹⁰ and R¹¹ together with the nitrogen atom to which they are bonded form a ring having 4, 5, 6 or more ring members.

18. The method as claimed in one or more of claims 1 to 17, in which aromatic carboxylic acid (I) and alcohol (II) are reacted in the molar ratio from 20:1 to 1:20, in each case based on the mole equivalents of carboxyl and hydroxyl groups.

19. The method as claimed in one or more of claims 1 to 18, which is carried out in the presence of homogeneous catalysts, heterogeneous catalysts or mixtures thereof.

## Revendications

1. Procédé continu pour la production d'esters d'acides carboxyliques aromatiques, dans lequel on fait réagir au moins un acide carboxylique aromatique de formule (I)
Ar-COOH (I)
dans laquelle Ar représente un radical aryle éventuellement substitué ayant de 5 à 50 atomes de carbone,
avec au moins un alcool de formule (II)
R²-(OH)ₙ (II)
dans laquelle
R² représente un radical hydrocarboné éventuellement substitué ayant de 1 à 100 atomes de carbone et
n représente un nombre valant de 1 à 10,
en présence d'au moins un catalyseur d'estérification, sous irradiation par micro-ondes dans un tube de réaction, dont l'axe longitudinal se trouve dans la direction de propagation des micro-ondes d'un applicateur de micro-ondes monomodal, pour l'obtention de l'ester, dans lequel l'irradiation du mélange réactionnel avec les micro-ondes s'effectue dans un tube de réaction essentiellement transparent aux micro-ondes à l'intérieur d'un guide tubulaire raccordé par guide d'onde à un générateur de micro-ondes.

2. Procédé selon la revendication 1, dans lequel l'applicateur de micro-ondes est conçu sous forme de résonateur à cavité.

3. Procédé selon la revendication 1 et/ou 2, dans lequel l'applicateur de micro-ondes est conçu sous forme de résonateur à cavité du type à réflexion.

4. Procédé selon une ou plusieurs des revendications 1 à 3, dans lequel le tube de réaction est aligné axialement avec un axe de symétrie central du guide tubulaire.

5. Procédé selon une ou plusieurs des revendications 1 à 4, dans lequel l'irradiation du mélange réactionnel s'effectue dans un résonateur à cavité à passage coaxial des micro-ondes.

6. Procédé selon une ou plusieurs des revendications 1 à 5, dans lequel on fait fonctionner le résonateur à cavité en mode E₀₁ₙ, n étant un nombre entier valant de 1 à 200.

7. Procédé selon une ou plusieurs des revendications 1 à 6, dans lequel une onde stationnaire se forme dans le résonateur à cavité.

8. Procédé selon une ou plusieurs des revendications 1 à 7, dans lequel le produit en réaction est chauffé par l'irradiation micro-ondes à des températures comprises entre 150 et 500 °C.

9. Procédé selon une ou plusieurs des revendications 1 à 8, dans lequel l'irradiation micro-ondes s'effectue sous des pressions supérieures à la pression atmosphérique.

10. Procédé selon une ou plusieurs des revendications 1 à 9, dans lequel Ar est un système cyclique à conjugaisons accumulées, comportant (4n + 2) électrons π, n étant égal à 1, 2, 3, 4 ou 5.

11. Procédé selon une ou plusieurs des revendications 1 à 10, dans lequel Ar est un groupe alkylaryle comportant au moins un radical alkyle ayant de 1 à 50 atomes de carbone.

12. Procédé selon une ou plusieurs des revendications 1 à 11, dans lequel Ar porte deux ou plus de deux groupes hydroxy et/ou groupes hydroxyalkyle.

13. Procédé selon une ou plusieurs des revendications 1 à 10, dans lequel Ar est choisi parmi l'acide benzoïque, l'acide phtalique, l'acide isophtalique, l'acide téréphtalique, les isomères de l'acide naphtalènecarboxylique, de l'acide pyridinecarboxylique et de l'acide naphtalènedicarboxylique, l'acide trimellitique, l'acide trimésique, l'acide pyromellitique et l'acide mellitique, les isomères de l'acide méthoxybenzoïque, l'acide hydroxybenzoïque, l'acide hydroxyméthylbenzoïque, l'acide hydroxyméthoxybenzoïque, l'acide hydroxydiméthoxybenzoïque, l'acide hydroxyisophtalique, l'acide hydroxynaphtalènecarboxylique, l'acide hydroxypyridinecarboxylique, l'acide hydroxyméthylpyridinecarboxylique, l'acide hydroxyquinoléinecarboxylique, l'acide o-tolylique, l'acide m-tolylique, l'acide p-tolylique, l'acide o-éthylbenzoïque, l'acide m-éthylbenzoïque, l'acide p-éthylbenzoïque, l'acide o-propylbenzoïque, l'acide m-propylbenzoïque, l'acide p-propylbenzoïque, l'acide 3,4-diméthylbenzoïque et l'acide thiophènecarboxylique.

14. Procédé selon une ou plusieurs des revendications 1 à 13, dans lequel R² représente un radical aliphatique éventuellement substitué ayant de 2 à 24 atomes de carbone.

15. Procédé selon une ou plusieurs des revendications 1 à 13, dans lequel R² représente un groupe aryle en C₆-C₁₂ éventuellement substitué ou un groupe hétéroaromatique éventuellement substitué ayant de 5 à 12 chaînons formant le cycle.

16. Procédé selon une ou plusieurs des revendications 1 à 15, dans lequel n est égal à un, deux, trois, quatre, cinq ou six.

17. Procédé selon une ou plusieurs des revendications 1 à 13, dans lequel R² représente des radicaux de formule (III)
-(R⁴-O)ₙ-R⁵ (III)
dans laquelle
R⁴ représente un groupe alkylène ayant de 2 à 18 atomes de carbone, ou des mélanges de tels groupes,
R⁵ représente un atome d'hydrogène ou un radical hydrocarboné ayant de 1 à 24 atomes de carbone ou un groupe de formule -R⁴-NR¹⁰R¹¹,
n représente un nombre compris entre 1 et 500 et
R¹⁰, R¹¹ représentent indépendamment l' un de l'autre un radical aliphatique ayant de 1 à 24 atomes de carbone, un groupe aryle ou hétéroaryle ayant de 5 à 12 chaînons formant le cycle, un groupe poly(oxyalkylène) comportant de 1 à 50 unités poly(oxyalkylène), les unités polyoxyalkylène dérivant d'unités oxyde d' alkylène ayant de 2 à 6 atomes de carbone, ou R¹⁰ et R¹¹ représentent ensemble avec l'atome d'azote, auquel ils sont liés, un cycle ayant 4, 5, 6 ou plus de 6 chaînons formant le cycle.

18. Procédé selon une ou plusieurs des revendications 1 à 17, dans lequel on met en réaction l'acide carboxylique aromatique (I) et l'alcool (II) en un rapport molaire de 20:1 à 1:20, chaque fois sur la base des équivalents molaires des groupes carboxy et hydroxy.

19. Procédé selon une ou plusieurs des revendications 1 à 18, qui est effectué en présence de catalyseurs homogènes, de catalyseurs hétérogènes ou de mélanges de ceux-ci.
